# EUROPEAN PATENT SPECIFICATION

(11) **EP 1 972 688 B1**
(45) Date of publication and mention of the grant of the patent: **02.06.2010**
(21) Application number: 07114683.1
(22) Date of filing: 21.08.2007
(51) Int. Cl.: C12N 15/10, C12Q 1/68

(54) **Method of amplifying nucleic acid from microorganism using nonplanar solid substrate**
Verfahren zur Verstärkung von Nukleinsäure aus Zellen von Mikroorganismen mittels eines nonplanaren festen Substrats
Procédé d'amplification d'acide nucléique d'un microorganisme utilisant un substrat solide non planaire

(30) Priority: 21.08.2006 KR 20060079054; 21.08.2006 KR 20060079055; 21.08.2006 KR 20060079053; 21.08.2006 KR 20060079056
(43) Date of publication of application: 24.09.2008
(73) Proprietor: Samsung Electronics Co., Ltd., Suwon-si, Gyeonggi-do (KR)
(72) Inventor: Jeong, Sung-young, Gyeonggi-do (KR); Hwang, Kyu-youn, Gyeonggi-do (KR); Kim, Joon-ho, Gyeonggi-do (KR); Han, Jung-im, Gyeonggi-do (KR); Lee, Hun-joo, Gyeonggi-do (KR)
(74) Representative: Grünecker, Kinkeldey, Stockmair & Schwanhäusser Anwaltssozietät

(56) References cited:
- WO-A-95/23872
- WO-A-98/51693
- WO-A-2005/093065
- US-A- 5 085 779
- US-A1- 2004 241 462
- ENG J: "Effect of sodium polyanethol sulfonate in blood cultures" JOURNAL OF CLINICAL MICROBIOLOGY 1975, vol. 1, no. 2, 1975, pages 119-123, XP002490326 ISSN: 0095-1137
- SE HO PARK ET AL: "Cylindrical pillars in silicon PCR chip enhance the performance of DNA amplification" SOLID-STATE SENSORS, ACTUATORS AND MICROSYSTEMS, 2005. DIGEST OF TECHN ICAL PAPERS. TRANSDUCERS '05. THE 13TH INTERNATIONAL CONFERENCE ON SEOUL, KOREA JUNE 5-9, 2005, PISCATAWAY, NJ, USA,IEEE, vol. 2, 5 June 2005 (2005-06-05), pages 1604-1607, XP010828796 ISBN: 978-0-7803-8994-6

## Description

### BACKGROUND OF THE INVENTION

### 1. Field of the Invention

The present invention relates to a method of amplifying a nucleic acid from a microorganism using a nonplanar solid substrate.

### 2. Description of the Related Art

In conventional methods of amplifying nucleic acids from microorganisms, microorganism separation, nucleic acid isolation and nucleic acid amplification are separately performed. Microorganism separation is performed using centrifugal separation and filtration.

Conventional methods of purifying nucleic acids using a solid phase are known. For example, U.S. Patent No. 5,234,809 discloses a method of purifying nucleic acids using a solid phase to which nucleic acids are bound. However, this method is time-consuming and complicated, and thus is not suitable for a Lab-On-a-Chip (LOC). Also, a chaotropic salt material must be used in this method, because nucleic acids are not bound to the solid phase in the absence of a chaotropic salt material.

U.S. Patent No. 6,291,166 discloses a method of archiving nucleic acids using a solid phase matrix. This method is advantageous in that nucleic acids are irreversibly bound to the solid phase matrix, so that a delayed analysis or repeated analysis of the nucleic acid solid phase matrix complexes is possible. However, according to this method, alumina, which has a positively charged surface, should be rendered hydrophilic with basic materials, such as NaOH. Thereby, nucleic acids are irreversibly bound to the hydrophilic alumina, and thus cannot be separated from the alumina.

US Patent No. 5,705,628 discloses a method of irreversibly and non-specifically binding DNA in a DNA-containing solution, which has been mixed with a salt and polyethylene glycol, to a magnetic microparticle having a carboxyl group-coated surface. This method uses a magnetic microparticle having a carboxyl group-coated surface, a salt, and polyethylene glycol, in order to isolate DNA.

As described above, conventional methods of isolating and purifying a nucleic acid require the addition of a high-concentration reagent for DNA binding. However, such addition can affect subsequent processes, such as a polymerase chain reaction (PCR) and cannot be performed on a lab-on-a-chip (LOC). In addition, conventional methods of isolating and purifying a nucleic acid are performed independently and spatially separated from a method of purifying or concentrating a cell or virus. A method of performing isolation of the microorganism and amplification of a nucleic acid from the microorganism in a single vessel is not known.

Accordingly, there is a need to develop a method of amplifying a nucleic acid from a microorganism using a solid substrate, in which method a microorganism cell is isolated or concentrated by binding the microorganism cell to a solid surface, such as a substrate, and back-to-back, a nucleic acid derived from the microorganism cell can be purified and concentrated.

In addition, in a conventional method of detecting a microorganism from blood, there are many cases where the concentration of the microorganism is so low that the microorganism cannot be directly detected. To solve this problem, a method of growing the microorganism by culturing the blood has been used. In general, a medium including sodium polyanethol sulfonate (SPS) is used in the blood culture (for example, Blood Culture Bottle (BC), see http://www.hylabs.co.il/SiteFiles/1/36/180.asp). SPS is a polyanion anticoagulant used for survival of bacteria cells in blood culture. SPS has a structure similar to that of a nucleic acid base, and thus, SPS acts as an intercalator to inhibit PCR. In addition, the SPS is not removed by a nucleic acid isolation kit, for example, a QlAgen kit, which is commercially available. Therefore, when a nucleic acid from a conventional sample including SPS, for example, a blood culture including SPS, is amplified by performing PCR, the sample has to be diluted more than about 5,000 times. In addition, SPS has to be separately removed from the nucleic acid that is isolated using a conventional nucleic acid isolation kit, which is commercially available.

### SUMMARY OF THE INVENTION

The present invention provides a method of amplifying nucleic acids from microorganisms using a nonplanar solid substrate.
According to an aspect of the present invention, there is provided a method of amplifying a nucleic acid from a microorganism comprising: contacting a microorganism-containing sample with a nonplanar solid substrate in a liquid medium having a pH in a range of pH 3.0 to pH 6.0 and attaching the microorganism to the nonplanar solid substrate;
washing the nonplanar solid substrate to remove unbound materials that are not attached thereto; and
performing PCR using the microorganism attached to the nonplanar solid substrate as a template sample and amplifying nucleic acid from the microorganism, wherein the contacting, attaching, washing and PCR are performed in the same vessel, and wherein the micro-organism containing sample comprises sodium polyanethol sulfonate.

### BRIEF DESCRIPTION OF THE DRAWINGS

The above and other features and advantages of the present invention will become more apparent by describing in detail exemplary embodiments thereof with reference to the attached drawings in which:
FIG. 1 is a graph showing the results of a PCR performed by attaching *E*. *coli* to a nonplanar solid substrate and then using DNA of the attached *E*. *coli* as a template, according to an embodiment of the present invention;
FIGS. 2A and 2B are graphs showing the results of a PCR performed after a blood or serum sample that each includes *E. coli* is attached to a nonplanar solid substrate, and the cells are disrupted by heat, according to an embodiment of the present invention;
FIG. 3 is a graph showing the results of a PCR performed after DNA is isolated using a nonplanar solid substrate and a QIAGEN kit, respectively, wherein the PCR results are represented as Ct values, according to an embodiment of the present invention;
FIG. 4 illustrates the electrophoresis results of PCR products in the case of performing PCR after DNA is isolated using a nonplanar solid substrate and a QIAGEN kit, respectively, according to an embodiment of the present invention;
FIG. 5 is a graph showing the results of cell disruption and nucleic acid amplification performed after *E. coli* is separated by contacting blood or urine samples including *E*. *coli* with a nonplanar solid substrate having a surface that is coated with OTC, according to an embodiment of the present invention;
FIGS 6 and 7 are graphs showing the results of a spectroscopic analysis of a solution containing SPS in the case of before (FIG. 6) and after (FIG. 7) the solution containing SPS is flowed through a fluidic device including a nonplanar solid substrate according to an embodiment of the present invention;
FIG. 8 illustrates the results of a real-time PCR performed by separating *E. coli* cells from a blood culture including SPS and a blood culture excluding SPS using a fluidic device including a nonplanar solid substrate and then disrupting the *E. coli* cells, and using DNA from the disrupted cells as a template;
FIG. 9 illustrates the electrophoresis results of a PCR performed by separating *E. coli* cells from a blood culture including SPS and a blood culture excluding SPS using a fluidic device including a nonplanar solid substrate and then disrupting the *E. coli* cells, and using the DNA from the disrupted cells as a template in the PCR process; and
FIG. 10 illustrates the electrophoresis results of performing PCR using various DNA samples that include SPS or exclude SPS as a template and performing electrophoresis for the amplified products.

### DETAILED DESCRIPTION OF THE INVENTION

A method of amplifying a nucleic acid from a microorganism, according to an embodiment of the present invention, includes contacting a microorganism-containing sample with a nonplanar solid substrate in a liquid medium having a range of pH 3.0 to pH 6.0 and attaching the microorganism to the nonplanar solid substrate.

In embodiments of the present invention, "nucleic acid" may refer to DNA, RNA and PNA, preferably, DNA.

In a system comprising a liquid medium that is in contact with a solid substrate, a microorganism such as bacteria are present in the liquid medium or can be attached to the solid substrate. It is known that such a distribution of the microorganism is determined by the difference between the surface tension of the liquid medium and the surface tension of a microorganism. That is, when the surface tension of the liquid medium is greater than the surface tension of the microorganism, the microorganism is more easily attached to a solid substrate having a low surface tension, that is, a hydrophobic solid substrate. When the surface tension of the microorganism is greater than the surface tension of the liquid medium, the microorganism is more easily attached to a solid substrate having a high surface tension, that is, a hydrophilic solid substrate. When the surface tension of the microorganism is nearly the same as the surface tension of the liquid medium, it is reported that the surface tension does not affect the attachment of microorganism cells to a solid substrate, and other interactions such as an electrostatic interaction affect the attachment of a microorganism to a solid substrate (Applied and Environmental Microbiology, July 1983, p.90-97). It is known that, apart from the thermodynamic approach based on the surface tension, the microorganism can be attached to the surface of the solid substrate by electrostatic attractive force in addition to the surface tension. However, the binding velocity of the microorganism onto the solid substrate is very slow.

Therefore, to address these problems, the inventors of the present invention found that a large amount of microorganism cells can be separated by contacting a microorganism-containing sample with a nonplanar solid substrate in a liquid medium having a pH range of pH 3.0 to pH 6.0. The improved binding of a microorganism to a solid substrate may result from the increased surface area of a nonplanar solid substrate in relation to a planar substrate. Further, using a liquid medium of pH 3.0 to pH 6.0 can disrupt a cell membrane of a microorganism cell, and thus, the solubility of a microorganism cell in the liquid medium is lowered and more microorganism cells tend to bind to the surface of the nonplanar solid substrate. However, the present invention is not limited to such a mechanism.

In the contacting process, the microorganism-containing sample can be any sample containing a microorganism. In the present invention, the term "biological sample" denotes a sample that includes or is formed of a cell or tissue, such as a cell or biological liquid isolated from an individual. The individual can be an animal such as a human. The biological sample can be saliva, sputum, blood, blood culture, blood cells (for example, red blood cells or white blood cells), amniotic fluid, serum, sperm, bone marrow, tissue or micro needle biopsy sample, urine, peritoneum fluid, pleura fluid, or cell cultures. In addition, the biological sample can be a tissue section, such as a frozen section taken for a histological object. Preferably, the biological sample is a clinical sample derived from a human patient. More preferably, the biological sample is blood, blood culture, urine, saliva, or sputum. The biological sample can be a biological sample including sodium polyanethol sulfonate (SPS), for example, a blood culture including SPS.

SPS is a polyanion anticoagulant used for ensuring the survival of bacteria cells in blood culture. SPS has a structure similar to that of a nucleic acid base, and thus, SPS acts as an intercalator, which inhibits PCR. In addition, a nucleic acid isolation kit, for example, a commercially available QIAgen kit, cannot remove the SPS.

According to an embodiment of the present invention, a microorganism cell that is to be isolated can be a bacterial cell, fungus, or a virus.

In the contacting process, the biological sample can be diluted in the liquid medium, which may preferably be a solution or buffer that may buffer the microorganism cell in an environment having a low pH. The buffer can be a phosphate buffer, such as sodium phosphate having a pH of 3.0 to 6.0, or an acetate buffer, such as sodium acetate having a pH of 3.0 to 6.0. The degree of dilution is not limited, and the biological sample can be diluted in the liquid medium in a ratio of 1:1 to 1:1,000, and preferably, 1:1 to 1:10.

In the contacting process, the liquid medium may have a salt concentration of 10 mM to 500 mM, and preferably, 50 mM to 300 mM. That is, the biological sample may contact the nonplanar solid substrate in a liquid medium that may have an acetate or phosphate ion concentration of 10 mM to 500 mM, preferably 50 mM to 300 mM. The above described preferred pH-range and salt concentration refers to the pH and salt concentration of the liquid medium containing the sample present in the contacting step.

In the contacting process, the solid substrate has a nonplanar shape so that the surface area of the solid substrate can be increased compared to a planar substrate. The nonplanar solid substrate may have a corrugated surface. In the present invention, a corrugated surface denotes a non-level surface having grooves and ridges. The corrugated surface can be a surface with a plurality of pillars or a sieve-shaped surface with a plurality of pores. However, the corrugated surface may have other shapes.

In the contacting process, the nonplanar solid substrate may have various shapes. For example, the nonplanar solid substrate may be a solid substrate having a surface with a plurality of pillars, a bead-shaped solid substrate, or a sieve-shaped solid substrate having a plurality of pores in its surface. The solid substrate can be a single solid substrate or a combination of solid substrates, such as a solid substrate assembly which fills a tube or container.

In the contacting process, the nonplanar solid substrate may form an inner wall of a microchannel or microchamber of a microfluidic device. Accordingly, the method of amplifying nucleic acid from a microorganism according to the current embodiment of the present invention can be used in a fluidic device or microfluidic device having at least one inlet and outlet in fluid communication with a channel or microchannel.

As used herein, the term "microfluidic device" incorporates the concept of a microfluidic device that comprises microfluidic elements such as, e.g., microfluidic channels (also called microchannels or microscale channels). As used herein, the term "microfluidic" refers to a device component, e.g., chamber, channel, reservoir, or the like, that includes at least one cross-sectional dimension, such as depth, width, length, diameter, etc. of from about 0.1 micrometer to about 1000 micrometer.
Thus, the term "microchamber" and "microchannel" refer to a channel and a chamber that includes at least one cross-sectional dimension, such as depth, width, and diameter of from about 0.1 micrometer to about 1000 micrometer, respectively.

In the method of amplifying a nucleic acid from a microorganism according to the current embodiment of the present invention, in the contacting process, the nonplanar solid substrate may have a surface having a plurality of pillars. A method of forming pillars on a solid substrate is well known in the art. For example, micro pillars can be formed in a high density structure using a photolithography process used in a semiconductor manufacturing process. The pillars may have an aspect ratio, the height of the pillar : the length of the cross section of 1:1-20:1, but the aspect ratio is not limited thereto. The term "aspect ratio" used herein refers to a ratio of a height of a pillar to the length of a cross section of a pillar . The length of a cross section of the pillar refers to a diameter when the shape of the cross section is a circle and it refers to an average value of the length of each side when the shape of the cross section is a rectangle. In the pillar structure, the ratio of the height of the pillars to the distance between adjacent pillars may be in the range of 1:1 to 25:1.

The distance between adjacent pillars may be in the range of 5-100 µm.

In the contacting process, the nonplanar solid substrate or a surface thereof may be hydrophobic and have a water contact angle of 70° to 95°. In a nonplanar solid substrate may have a surface having a plurality of pillars, for example, the surface having a plurality of pillars may be hydrophobic. The hydrophobicity may be provided by coating a surface or defined areas of the non-planar solid support with a compound selected from the group consisting of octadecyldimethyl (3-trimethoxysilyl propyl)ammonium (OTC),
tridecafluorotetrahydrooctyltrimethoxysilane (DFS), CF₃(CF₂)₃CH₂CH₂SI(OCH₃) ₃, CF₃(CF₂)₅CH₂CH₂SI(OCH₃)₃, CF₃(CF₂)₇CH₂CH₂SI(OCH₃)₃, CF₃ (CF₂)₉CH₂CH₂SI(OCH₃)₃, (CF₃)₂CF(CF₂)₄CH₂CH₂SI(OCH₃)₃, (CF₃) ₂CF(CF₂)₆CH₂CH₂SI(OCH₃)₃, (CF₃)₂CF(CF₂)₈CH₂CH₂SI(OCH₃)₃, CF₃ (C₆H₄)C₂H₄Si(OCH₃)₃, CF₃ (CF₂)₃(C₆H₄)C₂H₄Si(OCH₃)₃, CF₃ (CF₂)₅(C₆H₄)C₂H₄Si(OCH₃)_{3,} CF₃(CF₂)₇(C₆H₄)C₂H4Si(OCH₃)₃, CF₃(CF₂) ₃CH₂CH₂SiCH₃(OCH₃)₂, CF₃(CF₂)₅CH₂CH₂SiCH₃(OCH₃)₂, CF₃ (CF₂)₇CH₂CH₂SiCH₃(OCH₃)₂, CF₃(CF₂)₉CH₂CH₂SiCH₃(OCH₃)₂, (CF₃) ₂CF(CF₂)₄CH₂CH₂SiCH₃(OCH₃)₂, (CF₃)₂CF(CF₂)₆CH₂CH₂SiCH₃(OCH₃)₂, (CF₃) ₂CF(CF₂)₈CH₂CH₂SiCH₃(OCH₃)₂, CF₃(C₆H₄)C₂H₄SiCH₃(OCH₃)₂, CF₃(CF₂)₃ (C₆H₄)C₂H₄SiCH₃(OCH₃)₂, CF₃(CF₂)₅(C₆H₄)C₂H₄SiCH₃(OCH₃)₂, CF₃ (CF₂)₇(C₆H₄)C₂H₄SiCH₃(_{O}CH₃)₂, CF₃(CF₂)₃CH₂CH₂Si(OCH₂CH₃)₃, CF₃ (CF₂)₅CH₂CH₂Si(OCH₂CH₃)₃, and CF₃ (CF₂)₇CH₂CH₂Si(OCH₂CH₃). For example, a SiO₂ layer of a solid support may be coated with a self-assembled monolayer (SAM) of a compound selected from the group consisting of OTC and DFS to provide a water contact angle of 70-95°.

In this application, the term "water contact angle" refers to water contact angle measured by a Kruss Drop Shape Analysis System type DSA 10 Mk2. A droplet of 1.5 µl deionized water is automatically placed on the sample. The droplet was monitored every 0.2 seconds for a period of 10 seconds by a CCD-camera and analyzed by Drop Shape Analysis software (DSA version 1.7, Kruss). The complete profile of the droplet was fitted by the tangent method to a general conic section equation. The angles were determined both at the right and left side. An average value is calculated for each drop and a total of five drops per sample are measured. The average of the five drops is taken the contact angle.

In the contacting process, the nonplanar solid substrate or a surface thereof may have at least one amine-based functional group at its surface. In a nonplanar solid substrate may have a surface having a plurality of pillars, for example, the surface having a plurality of pillars may have at least one amine-based functional group. The surface of the nonplanar solid substrate having at least one amine-based functional group may be prepared by coating the nonplanar solid substrate or a surface thereof with a compound selected from the group consisting of polyethyleneiminetrimethoxysilane (PEIM), aminopropyltriethoxysilane, N-(3-trimethoxysily)-propyl)ethylenediamine, and N-trimethoxysilylpropy-N,N,N-chloride trimethylammonium to provide a surface having the at least one amine-based functional group. For example, a SiO2 layer of a solid support may be coated with an SAM of PEIM. The amine-based functional group is positively charged at a pH of 3.0-6.0.

In the contacting process, the nonplanar solid substrate can be a substrate formed of any kind of material that has the water contact angle in the range described above and/or has at least one amine-based functional group at its surface. For example, the nonplanar solid substrate can be formed of glass, silicon wafer, plastic, or the like, but is not limited thereto. When a nonplanar solid substrate with a surface having a water contact angle of 70° to 95° and/or a surface having at least one amine-based functional group is contacted with a sample containing a microorganism cell, the microorganism cell in the liquid medium is assumed to be bound to the nonplanar solid substrate. However, the present invention is not limited to such a specific mechanism.

The method of amplifying a nucleic acid from a microorganism according to the current embodiment of the present invention may further include, after the contacting process, washing the nonplanar solid substrate and remove other materials, excluding the target microorganism cell, which are not bound to the nonplanar solid substrate, from the solid substrate. In the washing process, any solution that does not release the target microorganism cell bound to the nonplanar solid substrate from the nonplanar solid substrate and does remove impurities, which may adversely affect subsequent processes, can be used. For example, an acetate buffer or phosphate buffer which is used as a binding buffer can be used as the washing solution. The washing solution may have a pH of 3.0 to 6.0.

The method of amplifying a nucleic acid from a microorganism according to the current embodiment of the present invention includes amplifying a nucleic acid from the microorganism attached to the nonplanar solid substrate by performing PCR.

In the nucleic acid amplification, a nucleic acid can be amplified by arbitrary methods known to those of ordinary skill in the art, preferably by PCR. The term "PCR" denotes a polymerase chain reaction, a method of amplifying a target nucleic acid using a pair of primers that specifically bind to the target nucleic acid and using a polymerase. In general, PCR requires a template, a primer, a DNA polymerase, four types of DNA monomers such as dATP, dGTP, dCTP and dTTP, and a buffer. In the nucleic acid amplification, the DNA template may be a nucleic acid that is exposed at the surface of the microorganism attached to the nonplanar solid substrate by thermal cycling during PCR. Therefore, in the PCR process, for example, a target nucleic acid can be amplified by adding a reaction compositions of PCR excluding the DNA template to a vessel containing the nonplanar solid substrate having the microorganism attached thereto and performing PCR. During the thermal cycling of PCR, it is considered that cells are disrupted in the pre-denaturation and/or denaturation step of PCR. However, the present invention is not limited to such specific mechanisms. Therefore, in the nucleic acid amplification, a DNA template included within the microorganism attached to the nonplanar solid substrate may also be used in the PCR process without separately being isolated.

In the method of amplifying a nucleic acid from a microorganism according to the current embodiment of the present invention, the contacting, washing and amplification processes are performed in one and the same single vessel. The vessel can be, for example, a microchannel, a microchamber, a tube and the like, but is not limited thereto. Preferably, the vessel may be a microchamber installed in a microfluidic device, and may include a PCR device. The PCR device may comprise, for example, a heater, a cooler and a thermostat. Therefore, in the method of amplifying a nucleic acid from a microorganism according to the current embodiment of the present invention, nucleic acid extraction and nucleic acid amplification may be performed in the same microchamber.

### Examples

Hereinafter, the present invention will be described in further detail with reference to the following examples. These examples are for illustrative purposes only and are not intended to limit the scope of the present invention.

### Example 1: Separation of E. coli cells from blood, disruption and nucleic acid amplification of the E. coli cells, using a nonplanar solid substrate having a pillar structure

A sample, i.e. a liquid medium containing cells was flowed through a fluidic device including an inlet and outlet, and having a chamber including a nonplanar solid substrate having a pillar array formed on a chip having an area of 10 mm x 23 mm. Then, PCR was performed using DNA from cells bound to the nonplanar solid substrate as a template.

In the pillar array, the distance between pillars was 15 µm, the pillars had a height of 100 µm, and each of the pillars had a square-shaped cross-section, wherein each side of the square-shaped cross-section had a length of 25 µm. The surface of the nonplanar solid substrate having the pillar array formed thereon was formed of a SiO₂ layer.

A sample having a pH of 4.7 obtained by mixing blood containing *E*. *coli* (OD₆₀₀=0.1) in a concentration of about 10⁸ cells/ml with the same amount of an acetate buffer having a pH of 3.0 was used as the sample including cells.

The sample was introduced into the inlet of the fluidic device, flowed through the chamber and was discharged at the outlet of the fluidic device. 500 µℓ of the sample was flowed through fluidic device at a flow rate of 200 µℓ/minute. Then, the solution remaining in the chamber fluidic device was centrifugally separated and removed from the chamber, and 3.0 µℓ of a PCR reaction solution was introduced into the chamber.

The PCR reaction solution was prepared by mixing 1x PCR buffer (Solgent Co. Ltd., Korea), 200 µM of dNTP, 200 nM of each primer, 2.5 mM of MgCl₂, 1 mg of BSA, 5% PEG, 0.5x Sybr green, and 0.3 units of Taq polymerase to reach a total volume of 3.0 µℓ. Each of the primers had a nucleotide sequence of SEQ ID Nos:1 and 2. The fluidic device including the chamber was installed in a TMC-1 000 (manufactured by Samsung Techwin, Co., Ltd., Korea). Then, *E. coli* cells were disrupted by heat, repeating three times at 95 °C for 10 seconds and at 65 °C for 10 seconds. The fluidic device used in the current example was installed in a TMC-1000 in a module form capable of accommodating the fluidic device, and thus, PCR could be performed in the fluidic device.

Then, thermal cycling for carrying out the PCR was performed. Conditions of the thermal cycling were as follows: predenaturation at 94 °C for 10 seconds, 40 cycles of denaturation at 94 °C for 10 seconds, annealing at 62 °C for 10 seconds and extension at 72 °C for 10 seconds.

The concentration of the nucleic acid amplified during the PCR process was determined by detecting SYBR™ green. The qPCR was conducted using a Lightcycler™ 2.0 Real-Time PCR system from Roche Company, and the Ct value was calculated automatically by using the method stored in the Lightcycler^{™} 2.0 Real-Time PCR system.

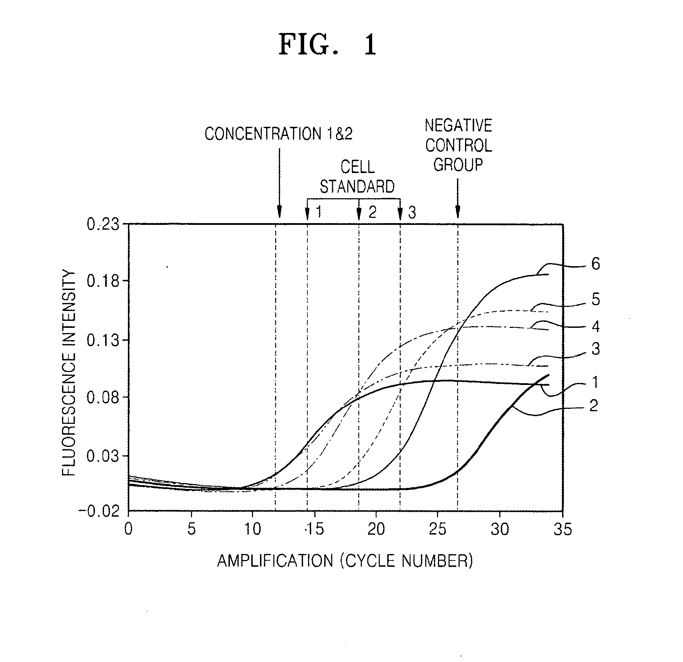

FIG. 1 is a graph showing the results of PCR performed by attaching *E*. *coli* to a nonplanar solid substrate and then using DNA from the attached *E*. *coli* as a template, according to an embodiment of the present invention. Ct values where obtained when a sample having 10⁷ *E. coli* cells /ml were concentrated using a nonplanar solid substrate (concentration Ct lines 1 and 2), and then the *E. coli* cells were disrupted by heat during a PCR process, and PCR was performed using DNA that was exposed from the cells disruption as a template. That is, in FIG. 1, curves 1 and 3 refer to a realtime PCR products curves for the samples 1 and 2 which is concentrated as mentioned above and the concentration Ct lines 1 and 2 refer to Ct lines for the curves 1 and 3. Curves 1 and 3 refer to results of 2 time repeats experiments under the same conditions. Further, Ct values were obtained when PCR was performed using the same process described above for a sample in which *E. coli* cells concentration by binding with a solid substrate did not occur (cell standard). In FIG. 1, curves 4-6 refer to a realtime PCR products curves for the standard samples 1-3 and the cell standard Ct lines 1-3 refer to Ct lines for the curves 4-6, respectively. The standard samples 1-3 refer to 10⁷ *E.coli* cells/reaction, 10⁶ *E.coli* cells/reaction, and 10⁵ *E.coli* cells/reaction, respectively. Curve 2 refers to a realtime PCR products curve for the negative sample, which does not contain a target DNA in PCR solution and the negative control group Ct line refers to Ct line for the curve 2. As can be seen from Fig. 1, the Ct value of "concentration 1 and 2" was lower than the Ct value of the "cell standard" experiment. This demonstrates that cell concentration was performed by the nonplanar solid substrate.

### Example 2: Separation of E. coli cells from blood and serum, and nucleic acid amplification from the E. coli cells using nonplanar solid substrate having a pillar structure: the effect of washing

A blood and a serum sample including cells was flowed through a fluidic device including an inlet and outlet, and having a chamber including a nonplanar solid substrate having a pillar array formed on a chip having an area of 10 mm x 23 mm to attach the cells to the nonplanar solid substrate. Then the nonplanar solid substrate was washed to remove materials that were not attached thereto, and PCR was performed using DNA from the cells attached to the nonplanar solid substrate as a template. In order to evaluate the effect of washing, the washing process of the nonplanar solid substrate was omitted in some of the experiments.

In the pillar array, the distance between pillars was 15 µm, the pillars had a height of 100 µm, and each of the pillars had a square-shaped cross-section, wherein each side of the square-shaped cross-section had a length of 25 µm. The surface of the nonplanar solid substrate having the pillar array formed thereon was formed of a SiO₂ layer.

Samples having a pH of 4.7 obtained by mixing each of blood and serum containing *E. coli* (OD₆₀₀=0.1) in a concentration of about 10⁸ cells/ml with the same amount of an acetate buffer having a pH of 3.0 were used as each of the blood and serum sample containing cells, respectively.

The blood and serum samples were introduced into the inlet of the fluidic device, flowed through the chamber and discharged at the outlet of the fluidic device. 500 µℓ of the blood and serum samples were flowed through the fluidic device at a flow rate of 200 µℓ/minute. When a washing process was performed, an acetate buffer having a pH of 4.0 was flowed through the fluidic device. Then, the solution remaining in the chamber of the fluidic device was centrifugally separated and removed, and 3.0 µℓ of a PCR reaction solution was introduced into the chamber.

The PCR reaction solution was prepared by mixing 1x PCR buffer, 200 µM of dNTP, 200 nM of each primer, 2.5 mM of MgCl₂, 1 mg of BSA, 5% PEG, 0.5x Sybr green, and 0.3 units of Taq polymerase to reach a total volume of 3.0 µl. Each of the primers had a nucleotide sequence of SEQ ID Nos: 1 and 2.

The fluidic device including the chamber was installed in a TMC-1 000 (manufactured by Samsung Techwin, Co., Ltd., Korea). Then, E. coli cells were disrupted by heat, repeating three times at 95 °C for 10 seconds and at 65 °C for 10 seconds. The fluidic device used in the current example was installed in a TMC-1000 in a module form, and thus, PCR could be performed in the fluidic device.

Then, thermal cycling for carrying out the PCR was performed. Conditions of the thermal cycling were as follows: predenaturation at 94 °C for 10 seconds, 40 cycles of denaturation at 94 °C for 10 seconds, annealing at 62 °C for 10 seconds and extension at 72 °C for 10 seconds.

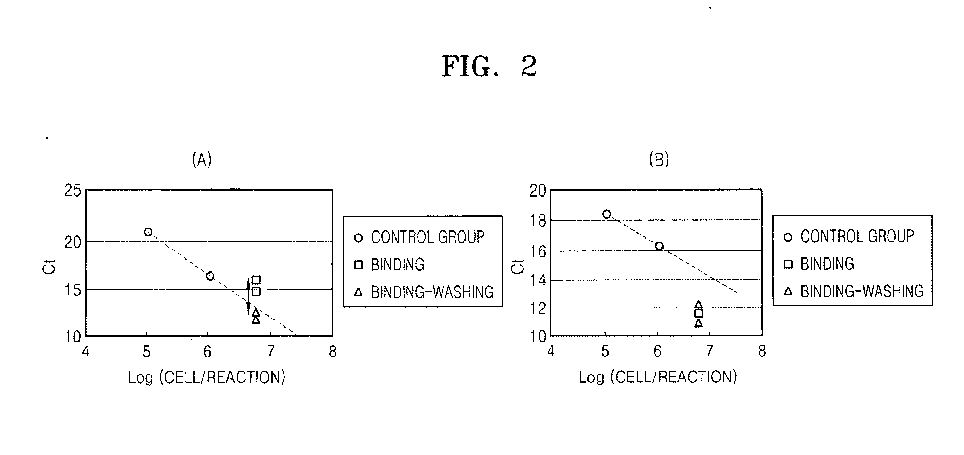

The concentration of the target nucleic acid amplified during the PCR process was determined by detecting SYBR™ green. The qPCR was conducted using a Lightcycler^{™} 2.0 Real-Time PCR system from Roche Company, and the Ct value was calculated automatically by using the method stored in the Lightcycler™ 2.0 Real-Time PCR system. FIGS. 2A and 2B are graphs showing the results of PCR performed using DNA from disrupted cells. The disrupted cells were obtained after a blood (Fig. 2A) or serum (Fig. 2B) sample that each contained *E. coli* was brought into contact with a solid substrate, cells attached to a solid substrate and the attached cells were disrupted by heat, according to an embodiment of the present invention.

Referring to FIGS. 2A and 2B, Ct values ("binding") were obtained when PCR was performed using DNA from disrupted cells as a template, wherein the *E*. *coli* cells were concentrated using a solid substrate and then disrupted. Ct values of a control group, performed in the same manner as the "binding" group, wherein the cell concentration was omitted, were lower than the Ct values of the "binding sample".

As illustrated in FIG. 2A referring to the blood sample, when washing the solid substrate to remove unbound materials that were not attached thereto, the Ct values of the blood sample obtained became lower compared to the "binding" sample. However, in the case of the serum sample (Fig. 2B), the washing process had no impact on the Ct values. As a result, it can be seen that a PCR inhibitor existed in the blood, and the washing process removed the PCR inhibitor.

In FIG. 2, a control was prepared by isolating DNA from blood using a QiAamp DNA mini kit, and performing PCR for the isolated DNA in the same manner as described above. The term "binding" related to a sample, in which the washing process is not performed after the binding process. The term "binding-washing" represents a sample in which the washing process is performed after the binding process. In FIG. 2A, an arrow represents the Ct values reduction according to washing. In FIG. 2, the cell concentration for the "binding" group and "binding washing" group at x axis refers to the initial celll concentration prior to subsequent cell concentration process.

### Example 3: Separation of E. coli cells from blood, disruption and nucleic acid amplification from the E. coli cells using nonplanar solid substrate having a pillar structure: impact on the concentration of E. coli cells

A blood sample containing cells was flowed through a fluidic device including an inlet and outlet, and having a chamber including a nonplanar solid substrate having a pillar array formed on a chip having an area of 10 mm x 23 mm to attach the cells to the nonplanar solid substrate. Then, the nonplanar solid substrate was washed to remove materials that were not attached thereto. Thereafter, the cells attached to the nonplanar solid substrate were disrupted by heat to obtain a cell lysate, and PCR was performed using DNA from the cell lysate as a template.

In the pillar array, the distance between pillars was 15 µm, the pillars had a height of 100 µm, and each of the pillars had a square-shaped cross-section, wherein each side of the square-shaped cross-section had a length of 25 µm. The surface of the nonplanar solid substrate having the pillar array formed thereon was formed of a SiO₂ layer.

Samples having a pH of 4.7, which were obtained by mixing blood containing *E*. *coli* (OD₆₀₀=0.01) in a concentration of about 10⁷ cells/ml and blood containing E. coli (OD₆₀₀=0.001) in a concentration of about 10⁶ cells/ml with the same amount of an acetate buffer having a pH of 3.0, were used as the blood sample containing cells.

The blood sample was introduced into the inlet of the fluidic device, flowed through the chamber and discharged at the outlet of the fluidic device. 500 µℓ of the blood sample was flowed through the fluidic device at a flow rate of 200 µℓ/minute. Then, an acetate buffer having a pH of 4.0 was flowed through the chamber. 500 µℓ of the acetate buffer was conveyed through the fluidic device at a flow rate of 200 µℓ/minute. Thereafter, the solution remaining in the chamber in the fluidic device was centrifugally separated and removed, and 3.5 µℓ of a PCR reaction solution was introduced into the chamber (experimental group).

The PCR reaction solution was prepared by mixing 1x PCR buffer, 200 µM of dNTP, 900 nM of each primer, 2.5 mM of MgCl₂, 1 mg of BSA, 5% PEG, 400 nM of Taqman probe (SEQ ID No: 3) and 0.1 units of Taq polymerase to reach a total volume of 3.5 µℓ. Each of the primers had a nucleotide sequence of SEQ ID Nos: 1 and 2. As a comparative experiment, DNA was isolated from the blood sample including 10⁶ cells/ml and 10⁷ cells/ml, respectively using a QIAamp DNA mini kit (manufactured by QIAGEN), and PCR was performed using the isolated DNA as a template in the same manner described above (comparative experimental group).

The fluidic device including the chamber was installed in a TMC-1000 (manufactured by Samsung Techwin, Co., Ltd., Korea). Then, E. coli cells were disrupted by heat, repeating three times at 95 °C for 10 seconds and at 65 °C for 10 seconds. The fluidic device used in the current example was installed in a TMC-1000 in a module form, and thus PCR could be performed in the fluidic device.

Then, thermal cycling for carrying out the PCR was performed after the cell disruption process. Conditions of the thermal cycling were as follows: predenaturation at 94 °C for 10 seconds, 40 cycles of denaturation at 94 °C for 5 seconds, annealing at 45 °C for 20 seconds and extension at 72 °C for 20 seconds.

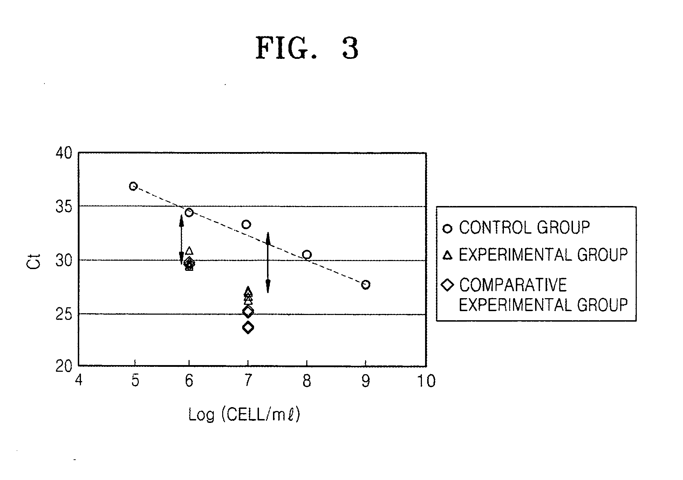

The concentration of the target nucleic acid amplified during the PCR process was determined by detecting SYBR™ green. The qPCR was conducted using a Lightcycler^{™} 2.0 Real-Time PCR system from Roche Company, and the Ct value was calculated automatically by using the method stored in the Lightcycler™ 2.0 Real-Time PCR system. FIG. 3 is a graph showing the results of PCR performed after DNA is isolated using a nonplanar solid substrate (experimental group) and a QIAGEN kit (comparative experimental group), respectively, wherein the PCR results are represented as Ct values, according to an embodiment of the present invention. An experiment for a control group was performed by supplying cells suspension having a different cell concentration that is, 10⁵ cells/ml, 10⁶ cells/ml, 10⁷ cells/ml, 10⁸ cells/ml, and 10⁹ cells/ml to the chamber without performing cell concentration. As illustrated in FIG. 3, the concentration effect of cells was shown for the experimental group and for the comparative experimental group, i.e. regardless of the method of concentrating the *E*. *coli* cells. In FIG. 3, the cell concentration for the experimental group and comparative experimental group at x axis refers to the initial celll concentration prior to the cell concentration process. The Ct values for the experimental group and comparative experiment group are shown as Table 1 below.

**Table 1.**

| | Initial cell concentration (cells/ml) | Ct values |
|---|---|---|
| Experimental group | 10⁶ | 29.64,31.00,29.54,29.70 |
| | 10⁷ | 27.73,26.3,27.13,26.7 |
| Comparative experimental group | 10⁶ | 29.5, 29.9 |
| | 10⁷ | 25.9,23.64,23.70,25.61 |

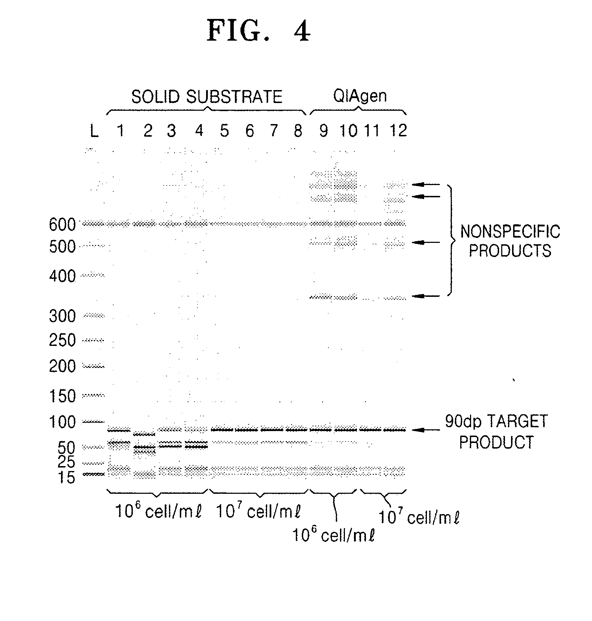

FIG. 4 illustrates the electrophoresis results of the PCR products in the case of performing PCR after DNA isolation using a nonplanar solid substrate and a QIAGEN kit, respectively, according to an embodiment of the present invention. As illustrated in FIG. 4, when a QIAGEN kit was used, nonspecific products were present in PCR products. However, when PCR was performed after DNA isolation using the nonplanar solid substrate, the nonspecific products were not detected in the electrophoresis gel. Therefore, when the method of amplifying a nucleic acid from a microorganism according to an embodiment of the present invention is used, specificity of nucleic acid amplification is high.

In FIG. 4, lanes 1-4 refer to results of an electrophoretic analysis of PCR products obtained from PCR amplifying DNA isolated using a nonplanar solid substrate and a sample including *E. coli* having a concentration of 10⁶ cells/ml, which are 4 time repeats of same experiments. Lanes 5-8 refer to results of an electrophoretic analysis of PCR products obtained from PCR amplifying DNA isolated using a nonplanar solid substrate and a sample including *E*. *coli* having a concentration of 10⁷ cells/ml, which are 4 time repeats of same experiments. Lanes 9-10, and lanes 11-12 refer to results of an electrophoretic analysis of PCR products obtained from PCR amplifying DNA isolated using a QIAGEN™ kit and a sample including *E*. *coli* having a concentration of 10⁶ cells/ml and 10⁷ cells/ml, respectively, which are 2 time repeats of same experiments, respectively. L refers to a DNA ladder. In FIG. 4, the expected size of target product is 90 bp.

### Example 4: DNA amplification using a nonplanar solid substrate having a pillar structure: effect of nonplanar solid substrate surface

In the current Example, PCR was performed in a chamber comprising a nonplanar solid substrate by introducing DNA into the chamber of a fluidic device including an inlet and outlet, and having a chamber including a solid substrate having a pillar array formed on a chip having an area of 10 mm x 23 mm.

In the pillar array, the distance between pillars was 15 µm, the pillars had a height of 100 µm, and each of the pillars had a square-shaped cross-section, wherein each side of the square-shaped cross-section had a length of 25 µm. The surface of the nonplanar solid substrate having the pillar array formed thereon was formed of a SiO₂ layer, wherein the SiO₂ layer was coated with OTC and PEIM. 3.5 µℓ of a PCR reaction solution was supplied to the chamber. The PCR reaction solution was prepared by mixing 1x PCR buffer, 200 µM of dNTP, 900 nM of each primer, 2.5 mM of MgCl₂, 1 mg of BSA, 5% PEG, 400 nM of Taqman probe, 1 ng of bacteria genome DNA and 0.1 units of Taq polymerase to reach a total volume of 3.5 µℓ. Each of the primers had a nucleotide sequence of SEQ ID Nos: 1 and 2.

Then the fluidic device including the chamber was installed in a TMC-1 000 (manufactured by Samsung Techwin, Co., Ltd., Korea), and thermal cycling for PCR was performed. Conditions of the thermal cycling were as follows: predenaturation at 94 °C for 10 seconds, 40 cycles of denaturation at 94 °C for 5 seconds, annealing at 45 °C for 20 seconds and extension at 72 °C for 20 seconds.

The concentration of the nucleic acid amplified during the PCR process was determined by detecting a Taqman probe. The qPCR was conducted using a Lightcycler^{™} 2.0 Real-Time PCR system from Roche Company, and the Ct value was calculated automatically by using the method stored in the Lightcycler^{™} 2.0 Real-Time PCR system. The concentration and Ct values determined for the amplified products with respect to each sample are shown in Table 2 below.

**Table 2**

| solid substrate type | Ct value |
|---|---|
| SiO2_1 | 17.75 |
| SiO2_2 | 16.68 |
| OTC-1 | 16.79 |
| OTC-2 | 16.22 |
| PEIM_1 | 30.19 |
| PEIM_2 | 33 |

As shown in Table 2, a solid substrate coating with OTC did not affect the PCR process in relation to a SiO₂ substrate. But, a solid substrate coated with PEIM, resulted in a poor PCR performance indicated by the high Ct values. The high Ct values using a solid substrate coated with PEIM likely result from the positively-charged surface of the PEIM layer, but, the present invention is not limited to such a specific mechanism.

### Example 5: Separation of E. coli cells from blood or urine, disruption and nucleic acid amplification from the E. coli cells using a nonplanar solid substrate having a pillar structure: effect of sample types

In the current example, a blood and a urine sample containing cells was respectively flowed through a fluidic device including an inlet and outlet, and having a chamber including a nonplanar solid substrate having a pillar array formed on a chip having an area of 10 mm x 23 mm to attach the cells to the nonplanar solid substrate. Then, the nonplanar solid substrate was washed to remove materials that were not attached thereto. Thereafter, the cells attached to the nonplanar solid substrate were disrupted by heat to obtain cell lysate, and PCR was performed using DNA from the cell lysate as a template.

In the pillar array, the distance between pillars was 15 µm, the pillars had a height of 100 µm, and each of the pillars had a square-shaped cross-section, wherein each side of the square-shaped cross-section had a length of 25 µm. The surface of the nonplanar solid substrate having the pillar array formed thereon was formed of a SiO₂ layer, wherein the SiO₂ layer was coated with OTC.

A sample having a pH of 4.8, which was obtained by mixing blood containing *E*. *coli* (OD₆₀₀=0.01) in a concentration of about 10⁷ cells/ml and urine including *E*. *coli* (OD₆₀₀=0.01) in a concentration of about 10⁷ cells/ml with the same amount of an acetate buffer having a pH of 3.0, were used as the blood and urine sample containing cells, respectively.

500 µℓ of the blood or urine sample was flowed through the chamber of the microfluidic device at a flow rate of 200 µℓ/minute. Then, 500 µℓ of an acetate buffer having a pH of 4.0 was flowed through the chamber of the fluidic device at a flow rate of 200 µℓ/minute. Thereafter, the solution remaining in the chamber of the fluidic device was centrifugally separated and removed , and 3.5 µℓ of a PCR reaction solution was added to the chamber. The PCR reaction solution was prepared by mixing 1x PCR buffer, 200 µM of dNTP, 900 nM of each primer, 2.5 mM of MgCl₂, 1 mg of BSA, 5% PEG, 400 nM of Taqman probe and 0.1 units of Taq polymerase to reach a total volume of 3.5 µℓ. Each of the primers had a nucleotide sequence of SEQ ID Nos: 1 and 2. The fluidic device including the chamber was installed in a TMC-1000 (manufactured by Samsung Techwin, Co., Ltd., Korea). Then, *E. coli* cells were disrupted by heat, repeating three times at 95 °C for 10 seconds and at 65 °C for 10 seconds. The fluidic device used in the current example was installed in a TMC-1000 in a module form, and thus, PCR could be performed in the fluidic device.

Then, thermal cycling for carrying out the PCR was performed after the cell disruption process. Conditions of the thermal cycling were as follows: predenaturation at 94 °C for 10 seconds, 40 cycles of denaturation at 94 °C for 5 seconds, annealing at 45 °C for 20 seconds and extension at 72 °C for 20 seconds.

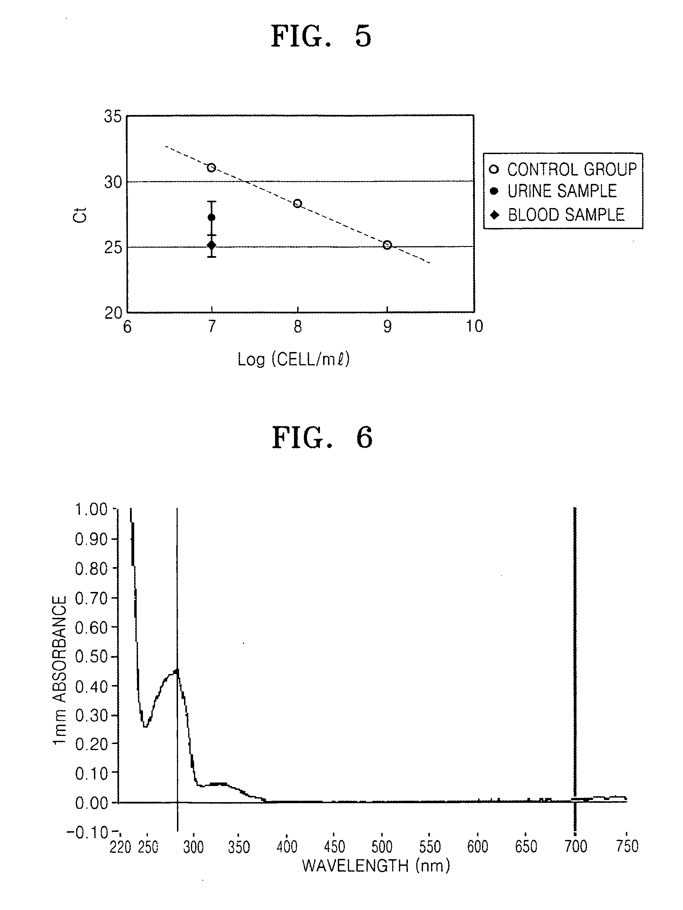

The concentration of the target nucleic acid amplified during the PCR process was determined by detecting Taqman probe. The qPCR was conducted using a Lightcycler™ 2.0 Real-Time PCR system from Roche Company, and the Ct value was calculated automatically by using the method stored in the Lightcycler™ 2.0 Real-Time PCR system. FIG. 5 is a graph showing the results of cell disruption and nucleic acid amplification performed after *E. coli* is separated by contacting the blood or the urine sample containing *E*. *coli* with a nonplanar solid substrate having a surface that is coated with OTC, according to an embodiment of the present invention. In a control group, PCR was performed in the same manner as described above except that DNA, which was isolated using a QIAGEN DNA mini kit (manufactured by QIAGEN), was used for PCR. As illustrated in FIG. 5, the blood and the urine sample resulted in a lower Ct value compared to the control group. From this result, it can be seen that cells in the blood or urine sample are concentrated, and cells in the blood and urine are concentrated by 117 times and 20.9 times, respectively. In FIG. 5, the Ct value for the blood sample is lower than that of urine sample. In FIG. 5, the cell concentration for the urine sample and blood sample at x axis refers to the initial cell concentration prior to subsequent cell concentration process.

### Example 6: Confirmation of whether sodium polyanethol sulfonate (SPS) is bound to a nonplanar solid substrate having a pillar structure

In the current example, a solution comprising sodium polyanethol sulfonate (SPS) was flowed through a fluidic device including an inlet and outlet, and having a chamber including a nonplanar solid substrate having pillar arrays formed on a chip having an area of 7.5 mm x 15 mm (Chip 1) and a chip having an area of 10 mm x 23 mm (Chip 2). A spectroscopic analysis was performed before and after the solution passed through the fluidic device in order to determine whether SPS was attached to the nonplanar solid substrate.

In the pillar arrays, the distance between pillars was 15 µm, the pillars had a height of 100 µm, and each of the pillars had a square-shaped cross-section, wherein each side of the square-shaped cross-section had a length of 25 µm. The surface of the nonplanar solid substrate having the pillar arrays formed thereon was formed of a SiO₂ layer.

A solution comprising 0.05 % SPS of a blood culture media solution was used as the solution including SPS. 500 µℓ of the solution was flowed through the fluidic device at a flow rate of 200 µℓ/minute.

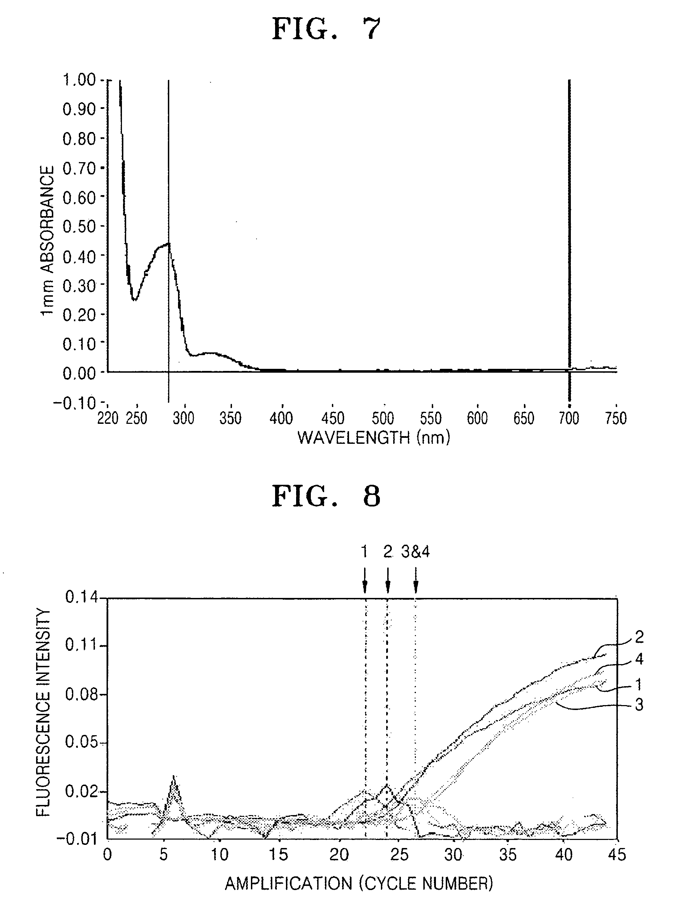

FIGS. 6 and 7 are graphs showing the results of the spectroscopic analysis before (FIG. 6) and after (FIG. 7) the solution containing SPS was flowed through the fluidic device including a nonplanar solid substrate, according to an embodiment of the present invention. In the spectroscopic analysis, a wavelength scan in the range of 220 nm to 750 nm was performed fluidic device, in particular, the chamber comprising the pillar structure, in order to determine the absorbance for each wavelength. As illustrated in FIGS. 6 and 7, the spectroscopic curve obtained before the solution was passed through the fluidic device principally corresponds to the spectroscopic curve obtained after the solution was passed through the fluidic device. The result represents that the SPS is not attached to the nonplanar solid substrate used in the current example. In chips 1 and 2, similar results were observed, and FIGS. 6 and 7 illustrate experimental results obtained using chip 1.

### Example 7: Amplification of a nucleic acid from a blood culture using a nonplanar solid substrate having a pillar structure: effect of SPS

In the current example, a blood culture containing *E. coli* and SPS was flowed through a fluidic device including an inlet and outlet, and having a chamber including a nonplanar solid substrate having a pillar array formed on a chip having an area of 10 mm x 23 mm to attach the *E. coli* cells to the nonplanar solid substrate. Then, the nonplanar solid substrate was washed to remove materials that were not attached thereto. Thereafter, the cells attached to the solid substrate were disrupted by heat to obtain cell lysate, and PCR was performed using DNA from the cell lysate as a template.

In the pillar array, the distance between pillars was 15 µm, the pillars had a height of 100 µm, and each of the pillars had a square-shaped cross-section, wherein each side of the square-shaped cross-section had a length of 25 µm. The surface of the nonplanar solid substrate having the pillar array formed thereon was formed of a SiO₂ layer.

A sample having a pH of 4.7, which was obtained by mixing a blood culture containing *E. coli* of 0.01 OD₆₀₀ and SPS with 100 mM of a sodium acetate buffer having a pH of 3.0 in a ratio of 1:1, was used as the blood culture sample flowed through the fluidic device. The blood culture was prepared by adding *E. coli* having a certain concentration to a BHI medium including 0.05 % of SPS and 10 % of blood. For the experiment, *E*. *coli* was added to the BHI medium in a predetermined concentration. In a control group, the same processes as described above were performed, except that a general blood culture excluding SPS was used. The general blood culture comprises a BHI medium excluding SPS and excluding blood.

The 500 µℓ of the blood culture sample was flowed through the chamber of the fluidic device at a flow rate of 200 µℓ/minute. Then, 500 µℓ of an acetate buffer having a pH of 4.0 was flowed through the chamber of the fluidic device at a flow rate of 500 µℓ/minute. Thereafter, the solution remaining in the chamber of the fluidic device was centrifugally separated and removed , and 3.5 µℓ of a PCR reaction solution was added to the chamber. The PCR reaction solution was prepared by mixing 1x PCR buffer, 200 µM of dNTP, 200 nM of each primer, 2.5 mM of MgCl₂, 1 mg of BSA, 5% PEG, 400 nM of Taqman probe (SEQ ID NO: 3) and 0.1 units of Taq polymerase to reach a total volume of 3.5 µℓ. Each of the primers had a nucleotide sequence of SEQ ID Nos: 1 and 2.

The fluidic device including the chamber was installed in a TMC-1 000 (manufactured by Samsung Techwin, Co., Ltd., Korea). Then, *E. coli* cells were disrupted by heat, repeating three times at 95 °C for 10 seconds and at 65 °C for 10 seconds. The fluidic device used in the current example was installed in a TMC-1000 in a module form, and thus PCR could be performed in the fluidic device.

Then, thermal cycling for carrying out the PCR was performed after the cell disruption process. Conditions of the thermal cycling were as follows: predenaturation at 94 °C for 10 seconds, 40 cycles of denaturation at 94 °C for 5 seconds, annealing at 45 °C for 20 seconds and extension at 72 °C for 20 seconds.

The concentration of the target nucleic acid amplified during the PCR process was determined by detecting a Taqman™ probe. The qPCR was conducted using a Lightcycler^{™} 2.0 Real-Time PCR system from Roche Company, and the Ct value was calculated automatically by using the method stored in the Lightcycler^{™} 2.0 Real-Time PCR system. FIG. 8 illustrates the results of real-time PCR performed after separating *E*. *coli* cells from a blood culture including SPS and a blood culture excluding SPS using a fluidic device including a nonplanar solid substrate and then disrupting the *E*. *coli* cells, and using DNA from the disrupted cells as a template for PCR. In FIG. 8, curves 1 and 2 refer to real-time PCR products curves for DNA obtained by separating E. coli cells from a blood culture excluding SPS, which are for the 2 time repeats experiments for the same conditions, and curves 3 and 4 refer to real-time PCR products curves for DNA obtained by separating E. coli cells from a blood culture including SPS, which are for the 2 time repeats experiments for the same conditions. The vertical Ct lines 1 and 2, 3 and 4 correspond to curves 1 and 2, and 3 and 4, respectively.

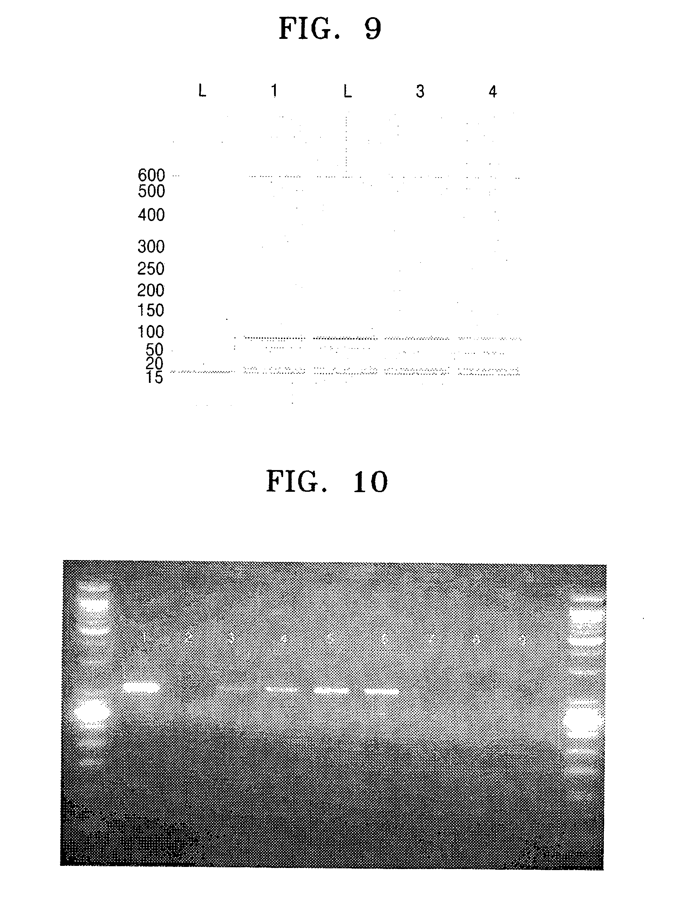

FIG. 9 illustrates the electrophoresis results obtained after separating *E*. *coli* cells from a blood culture including SPS and a blood culture excluding SPS using a fluidic device including a nonplanar solid substrate and then disrupting the E. coli cells, and using DNA from the disrupted cells as a template, and performing electrophoresis of the amplified PCR products. In FIG. 9, lane 1 refers to a DNA ladder, lanes 2 and 3 refer to a blood culture excluding SPS and lanes 3 and 4 refer to a blood culture including SPS. In FIG. 9, the expected size of target product is 90 bp. As illustrated in FIGS. 8 and 9, even when the blood culture including SPS was used as a sample, a target nucleic acid is amplified in a concentration similar to a concentration of a target nucleic acid amplified when the blood culture excluding SPS was used as a sample. The results show that a target nucleic acid can be amplified from a sample including SPS, without the requirement of separately removing SPS, in the method of amplifying nucleic acid from a microorganism according to an embodiment of the present invention.

### Example 8: Amplification of a nucleic acid from a sample including SPS using a nonplanar solid substrate having a pillar structure: effect of SPS

In the current example, a blood culture containing *E*. *coli* and SPS was flowed through a fluidic device including an inlet and outlet, and having a chamber including a solid substrate having a pillar array formed on a chip having an area of 10 mm x 23 mm to attach the *E*. *coli* cells to the nonplanar solid substrate. Then, the nonplanar solid substrate was washed to remove materials that were not attached thereto. Thereafter, the cells attached to the nonplanar solid substrate were disrupted in solution by a combined alkali and heat treatment to obtain a cell lysate, and PCR was performed using DNA from the cell lysate as a template.

In the pillar array, the distance between pillars was 15 µm, the pillars had a height of 100 µm, and each of the pillars had a square-shaped cross-section, wherein each side of the square-shaped cross-section had a length of 25 µm. The surface of the nonplanar solid substrate having the pillar array formed thereon was formed of a SiO₂ layer.

A sample having a pH of 4.7, which was obtained by mixing a blood culture containing *E. coli* of 0.01 OD₆₀₀ and SPS with 100 mM of a sodium acetate buffer having a pH of 3.0 in a ratio of 1:1, was used as the blood culture sample flowed through the fluidic device. The blood culture was prepared by adding *E. coli* to a BHI medium comprising 0.05 % of SPS and 10 % of blood. For the experiment, *E. coli* was added to the BHI medium in a predetermined concentration. A control group was prepared by performing the same processes as described above, except that a general blood culture excluding SPS was used. The general blood culture was a BHI medium excluding SPS and excluding blood.

500 µℓ of the blood culture sample was flowed through the chamber of the fluidic device at a flow rate of 200 µℓ/minute. Then, 500 µℓ of an acetate buffer having a pH of 4.0 was flowed through the chamber of the fluidic device at a flow rate of 500 µℓ/minute. Thereafter, the solution remaining in the chamber of the fluidic device was centrifugally separated and removed, and 0.01 N NaOH solution was introduced into the chamber. Then, the fluidic device was incubated at 95 °C for 5 minutes to disrupt the *E*. *coli* cells. The solution in the fluidic device was centrifugally removed and 2 ml of *E*. *coli* cell lysate was obtained from said solution.

As Comparative Experiments 3 and 4, 50 µℓ of a DNA solution are used, wherein the DNA was isolated, using a QiAgen mini kit, from a medium comprising 0.05% of SPS and 10% of blood and a medium excluding 0.05% of SPS and excluding 10% of blood.

In addition, as Comparative Experiments 1 and 2. 5 ng/µℓ of a DNA solution and a solution including 0.05 % of SPS and 5 ng/µℓ of DNA were used.

As Comparative Experiments 5 and 6, a PCR were performed using a medium including 0.05 % of SPS and 10 % of blood, and a medium excluding 0.05 % of SPS and 10 % of blood without isolating nucleic acid from the *E.coli* cells.

For PCR, a PCR reaction solution was added to a PCR tube. The PCR reaction solution was prepared by mixing 1x PCR buffer, 200 µM of dNTP, 200 nM of each primer, 2.5 mM of MgCl₂, 1 µℓ of each template and 2.5 units of Taq polymerase to reach a total volume of 50 µℓ. Each of the primers had a nucleotide sequence of SEQ ID Nos: 1 and 2. Thermal cycling for PCR was performed. Conditions of the thermal cycling were as follows: predenaturation at 95 °C for 1 minute, 25 cycles of denaturation at 95 °C for 5 seconds, annealing at 62 °C for 13 seconds and extension at 72 °C for 15 seconds.

FIG. 10 illustrates results of performing PCR using various DNA samples that include SPS or exclude SPS as a template and performing electrophoresis of the amplified products. In FIG. 10, Lanes 1 through 9 represent electrophoresis results of various DNA samples, the details are as follows:
Lane 1: DNA (Comparative Experiment 1),
Lane 2: DNA + 0.05% of SPS (Comparative Experiment 2),
Lane 3: a sample in which a nucleic acid is isolated from a culture medium comprising 0.05 % of SPS and 10 % of blood and E. coli of 0.005 OD600 using a QIAgen mini kit (Comparative Experiment 3),
Lane 4: a sample in which nucleic acid is isolated from a culture medium excluding 0.05 % of SPS and 10 % of blood and E. coli of 0.005 OD600 using a QIAgen mini kit (Comparative Experiment 4),
Lane 5: a sample in which a culture medium comprising 0.05 % of SPS and 10% of blood and E. coli of 0.005 OD600 is passed through a fluidic device including a nonplanar solid substrate according to an embodiment of the present invention, *E. coli* cells bound to the solid substrate disrupted, and then nucleic acid is eluted from the solid substrate (Experiment 1),
Lane 6: a sample in which a culture medium comprising and E. coli of 0.005 OD600 and excluding 0.05 % of SPS and 10% of blood is passed through a fluidic device including a solid substrate according to an embodiment of the present invention, E. coli cells bound to the solid substrate disrupted, and then nucleic acid is eluted from the solid substrate (Control Experiment),
Lane 7: a sample in which a culture medium including 0.05 % of SPS and 10 % of blood and E. coli of 0.005 OD600 is directly used for PCR (Comparative Experiment 5),
Lane 8; a sample in which a culture medium excluding 0.05 % of SPS and 10 % of blood and E. coli of 0.005 OD600 is directly used for PCR (Comparative Experiment 6), and
Lane 9: a negative control group which does not include a target DNA.

As illustrated in FIG. 10, it can be seen that SPS is a PCR inhibitor (from Lane 2). However, when E. coli cells are separated using a nonplanar solid substrate according to an embodiment of the present invention, the amplification of a nucleic acid is efficiently performed even if the sample comprises SPS (see lanes 5 and 6). Specifically, it was confirmed that the present method (lanes 5 and 6)_was more efficient that that of conventional method, that is, a method using a QIAGEN^{™} mini kit (lanes 3 and 4). Lanes 7 and 8 show that culture medium with or without SPS inhibits PCR. From the results, it is confirmed that in the process of separating *E. coli* cells using the nonplanar solid substrate according to an embodiment of the present invention, SPS is not attached to the nonplanar solid substrate, thereby being removed in the washing step.

In the method of amplifying a nucleic acid from a microorganism according to the present invention, microorganism separation, cell disruption and nucleic acid amplification are performed in a single vessel, and thus nucleic acid can be amplified conveniently, quickly and with high sensitivity. Also, these processes can be easily automated. In addition, since a microorganism can be contacted with a nonplanar solid substrate at a high flow rate in order to attach the microorganism to the nonplanar solid substrate, an initial sample with a large amount can be used.
## Claims

1. A method of amplifying a nucleic acid from a microorganism comprising: contacting a microorganism-containing sample with a nonplanar solid substrate in a liquid medium having a pH in a range of pH 3.0 to pH 6.0 and attaching the microorganism to the nonplanar solid substrate;
washing the nonplanar solid substrate and removing unbound materials that are not attached thereto; and
performing PCR using the microorganism attached to the nonplanar solid substrate as a template sample and amplifying a nucleic acid from the microorganism, wherein the contacting, attaching, washing and PCR are performed in the same vessel, and wherein the microorganism-containing sample comprises sodium polyanethol sulfonate.

2. The method according to claim 1, wherein the microorganism-containing sample is blood, blood culture, urine or saliva.

3. The method according to any of claims 1 to 2, wherein the microorganism is a bacterial cell, fungus or a virus.

4. The method according to any of claims 1 to 3, wherein the microorganism-containing sample is diluted in the liquid medium, which is a phosphate buffer or an acetate buffer.

5. The method according to claim 4, wherein the microorganism-containing sample is diluted in a ratio of 1:1 to 1:10.

6. The method according to any of claims 1 to 5, wherein the liquid medium has a salt concentration of 10 mM to 500 mM.

7. The method according to claim 6, wherein the liquid medium has a salt concentration of 50 mM to 300 mM.

8. The method according to any of claims 1 to 7, wherein the nonplanar solid substrate can be selected from the group consisting of a solid substrate having a surface with a plurality of pillars, a bead-shaped solid substrate, and a sieve-shaped solid substrate having a plurality of pores.

9. The method according to claim 8, wherein the pillars of a solid substrate having a surface with a plurality of pillars have an aspect ratio of 1:1 to 20:1, wherein the aspect ratio refers to a ratio of the cross-sectional diameter of a pillar to the height of a pillar.

10. The method according to claim 8 or 9, wherein a nonplanar solid substrate comprises a solid substrate having a surface with a plurality of pillars, wherein a ratio of the height of the pillars to the distance between adjacent pillars is in the range of 1:1 to 25:1.

11. The method according to any of claims 8 to 10, wherein a nonplanar solid substrate comprises a solid substrate having a surface with a plurality of pillars, wherein the distance between adjacent pillars is in the range of 5 µm to 100 µm.

12. The method according to any of claims 1 to 11, wherein the nonplanar solid substrate or at least one defined surface region thereof is hydrophobic having a water contact angle of 70° to 95°.

13. The method according to claim 12, wherein the surface or the at least one defined region of the nonplanar solid substrate is coated with octadecyldimethyl(3-trimethoxysilyl propyl)ammonium (OTC) or tridecafluorotetrahydrooctyltrimethoxysilane (DFS).

14. The method according to any of claims 1 to 13, wherein the surface or at least one defined surface region of the nonplanar solid substrate has at least one amine-based functional group.

15. The method according to claim 14, wherein the surface or at least one defined surface region of the nonplanar solid substrate is coated with polyethyleneiminetrimethoxysilane (PEIM).

## Patentansprüche

1. Verfahren zum Vervielfältigen einer Nukleinsäure von einem Mikroorganismus umfassend:
in Kontakt bringen einer einen Mikroorganismus-enthaltenden Probe mit einem unebenen, festen Träger in einem flüssigen Medium, das einen pH-Wert im Bereich von pH 3 bis pH 6 aufweist, und Anlagern des Mikroorganismus an den unebenen, festen Träger;
Waschen des unebenen, festen Trägers und Entfernen ungebundener Stoffe, die sich nicht an den Träger angelagert haben; und
Durchführen einer PCR unter Verwenden des Mikroorganismus, der an dem unebenen, festen Träger angelagert ist, als Matrize und Vervielfältigen einer Nukleinsäure von dem Mikroorganismus, wobei das Inkontaktbringen, das Anlagern, das Waschen und die PCR in demselben Gefäß durchgeführt werden, und wobei die Mikroorganismen-enthaltende Probe Natriumpolyanetholsulfonat aufweist.

2. Verfahren gemäß Anspruch 1, wobei die Mikroorganismus-enthaltende Probe Blut, eine Blutkultur, Urin oder eine Speichelflüssigkeit ist.

3. Verfahren gemäß einem der Ansprüche 1 bis 2, wobei der Mikroorganismus eine Bakterienzelle, ein Pilz oder ein Virus ist.

4. Verfahren gemäß einem der Ansprüche 1 bis 3, wobei die Mikroorganismus-enthaltende Probe in dem flüssigen Medium, das ein Phosphatpuffer oder ein Acetatpuffer ist, verdünnt ist.

5. Verfahren gemäß Anspruch 4, wobei die Mikroorganismus-enthaltene Probe in einem Verhältnis von 1:1 bis 1:10 verdünnt ist.

6. Verfahren gemäß einem der Ansprüche 1 bis 5, wobei das flüssige Medium eine Salzkonzentration von 10 mM bis 500 mM aufweist.

7. Verfahren gemäß Anspruch 6, wobei das flüssige Medium eine Salzkonzentration von 50 mM bis 300 mM aufweist.

8. Verfahren gemäß einem der Ansprüche 1 bis 7, wobei der unebene, feste Träger ausgewählt sein kann aus der Gruppe bestehend aus einem festen Träger, der eine Oberfläche mit einer Vielzahl von Säulen aufweist, einem kugelförmigen festen Träger, und einem siebförmigen festen Träger, der eine Vielzahl von Poren aufweist.

9. Verfahren gemäß Anspruch 8, wobei die Säulen auf einem festen Träger, der eine Oberfläche mit einer Vielzahl von Säulen aufweist, ein Seitenverhältnis von 1:1 bis 20:1 aufweist, wobei sich das Seitenverhältnis auf das Verhältnis von dem Querschnittsdurchmesser von einer Säule zu der Höhe von einer Säule bezieht.

10. Verfahren gemäß Anspruch 8 oder 9, wobei ein unebener, fester Träger einen festen Träger umfasst, der eine Vielzahl von Säulen auf einer Oberfläche aufweist, wobei das Verhältnis von der Höhe der Säulen zu dem Abstand zwischen benachbarten Säulen im Bereich von 1:1 bis 25:1 liegt.

11. Verfahren gemäß einem der Ansprüche 8 bis 10, wobei ein unebener, fester Träger einen festen Träger umfasst, der eine Vielzahl von Säulen auf einer Oberfläche aufweist, wobei der Abstand zwischen benachbarten Säulen im Bereich von 5 µm bis bis 100 µm beträgt.

12. Verfahren gemäß einem der Ansprüche 1 bis 11, wobei der unebene, feste Träger oder wenigstens ein definierter Oberflächenbereich des Trägers hydrophob mit einem Wasserkontaktwinkel von 70° bis 95° ist.

13. Verfahren gemäß Anspruch 12, wobei die Oberfläche oder der wenigstens eine definierte Bereich von dem unebenen, festen Träger mit Octadecyldimethyl(3-trimethoxysilylpropyl)ammonium (OTC) oder Tridecafluorotetrahydrooctyltrimethoxysilan (DFS) überzogen ist.

14. Verfahren gemäß einem der Ansprüche 1 bis 13, wobei die Oberfläche oder wenigstens ein bestimmter Oberflächenbereich von dem unebenen, festen Träger wenigstens eine Amin-basierte funktionelle Gruppe aufweist.

15. Verfahren gemäß Anspruch 14, wobei die Oberfläche oder wenigstens ein bestimmter Oberflächenbereich von dem unebenen, festen Träger mit Polyethylenimintrimethoxysilan (PEIM) beschichtet ist.

## Revendications

1. Procédé d'amplification d'un acide nucléique d'un micro-organisme consistant à :
mettre en contact un échantillon contenant un micro-organisme avec un substrat solide non plan dans un milieu liquide ayant un pH compris entre 3,0 et 6,0 et fixer le micro-organisme au substrat solide non plan ;
laver le substrat solide non plan et éliminer les matières non liées qui ne sont pas fixées à celui-ci ; et
réaliser une PCR en utilisant le micro-organisme fixé au substrat solide non plan comme échantillon matrice et amplifier un acide nucléique du micro-organisme,
où la mise en contact, la fixation, le lavage et la PCR sont réalisés dans le même récipient, et où l'échantillon contenant un micro-organisme contient du polyanétholsulfonate de sodium.

2. Procédé selon la revendication 1, dans lequel l'échantillon contenant un micro-organisme est du sang, une culture de sang, de l'urine ou de la salive.

3. Procédé selon l'une quelconque des revendications 1 à 2, dans lequel le micro-organisme est une cellule bactérienne, un champignon ou un virus.

4. Procédé selon l'une quelconque des revendications 1 à 3, dans lequel l'échantillon contenant un micro-organisme est dilué dans le milieu liquide, qui est un tampon phosphate ou un tampon acétate.

5. Procédé selon la revendication 4, dans lequel l'échantillon contenant un micro-organisme est dilué dans un rapport de 1:1 à 1:10.

6. Procédé selon l'une quelconque des revendications 1 à 5, dans lequel le milieu liquide a une concentration en sel de 10 mM à 500 mM.

7. Procédé selon la revendication 6, dans lequel le milieu liquide a une concentration en sel de 50 mM à 300 mM.

8. Procédé selon l'une quelconque des revendications 1 à 7, dans lequel le substrat solide non plan peut être choisi dans le groupe constitué d'un substrat solide ayant une surface dotée d'une pluralité de piliers, d'un substrat solide en forme de bille, et d'un substrat solide en forme de tamis ayant une pluralité de pores.

9. Procédé selon la revendication 8, dans lequel les piliers d'un substrat solide ayant une surface dotée d'une pluralité de piliers ont un rapport de forme de 1:1 à 20:1, le rapport de forme désignant le rapport du diamètre d'un pilier en coupe transversale à la hauteur d'un pilier.

10. Procédé selon la revendication 8 ou 9, dans lequel un substrat solide non plan comprend un substrat solide ayant une surface dotée d'une pluralité de piliers, où le rapport de la hauteur des piliers à la distance entre des piliers adjacents est compris entre 1:1 et 25:1.

11. Procédé selon l'une quelconque des revendications 8 à 10, dans lequel un substrat solide non plan comprend un substrat solide ayant une surface dotée d'une pluralité de piliers, où la distance entre des piliers adjacents se situe dans la plage de 5 µm à 100 µm.

12. Procédé selon l'une quelconque des revendications 1 à 11, dans lequel le substrat solide non plan ou au moins une région superficielle définie de celui-ci est hydrophobe, ayant un angle de contact avec l'eau de 70° à 95°.

13. Procédé selon la revendication 12, dans lequel la surface ou ladite au moins une région définie du substrat solide non plan est revêtue d'octadécyldiméthyl(3-triméthoxysilylpropyl)ammonium (OTC) ou de tridécafluorotétrahydrooctyltriméthoxysilane (DFS).

14. Procédé selon l'une quelconque des revendications 1 à 13, dans lequel la surface ou au moins une région superficielle définie du substrat solide non plan possède au moins un groupe fonctionnel à base d'amine.

15. Procédé selon la revendication 14, dans lequel la surface ou au moins une région superficielle définie du substrat solide non plan est revêtue de polyéthylèneiminetriméthoxysilane (PEIM).


<110> Samsung Electronics co. Ltd.
<120> Method of Amplifying Nucleic Acid from Microorganism using Nonplanar solid Substrate
<130> EP47579FZ106pau
<140> not yet assigned
   <141> herewith
<150> KR 10-2006-0079053
   <151> 2006-08-21
<150> KR 10-2006-0079054
   <151> 2006-08-21
<150> KR 10-2006-0079055
   <151> 2006-08-21
<150> KR 10-2006-0079056
   <151> 2006-08-21
<160> 3
<170> KopatentIn 1.71
<210> 1
   <211> 18
   <212> DNA
   <213> Artificial Sequence
<220>
   <223> primer 1
<400> 1
   tgtatgaaga aggcttcg 18
<210> 2
   <211> 19
   <212> DNA
   <213> Artificial Sequence
<220>
   <223> primer 2
<400> 2
   aaaggtatta actttactc 19
<210> 3
   <211> 20
   <212> DNA
   <213> Artificial Sequence
<220>
   <223> Taqman probe
<400> 3
   gtactttcag cggggaggaa 20